# EUROPÄISCHE PATENTSCHRIFT

(11) **EP 1 713 516 B1**
(45) Veröffentlichungstag und Bekanntmachung des Hinweises auf die Patenterteilung: **22.08.2007**
(21) Anmeldenummer: 05714909.8
(22) Anmeldetag: 27.01.2005
(51) Int. Cl.: A61K 51/04, A61K 51/08, A61K 51/12

(54) **VERFAHREN UND KIT ZUR HERSTELLUNG VON RHENIUM-188 MARKIERTEN PARTIKELN**
METHOD AND KIT FOR THE PRODUCTION OF PARTICLES LABELLED WITH RHENIUM-188
PROCEDE ET NECESSAIRE POUR PRODUIRE DES PARTICULES MARQUEES A L'AIDE DE RHENIUM 188

(30) Priorität: 29.01.2004 DE 102004005280
(43) Veröffentlichungstag der Anmeldung: 25.10.2006
(73) Patentinhaber: ROTOP Pharmaka AG, 01454 Radeberg (DE)
(72) Erfinder: WUNDERLICH, Gerd, 01259 Dresden (DE); DREWS, Antje, 01326 Dresden (DE)
(74) Vertreter: Uhlemann, Henry
(86) Internationale Anmeldenummer: PCT/DE2005/000140
(87) Internationale Veröffentlichungsnummer: WO 2005/072781

(56) Entgegenhaltungen:
- US-A- 5 403 573
- US-A- 5 985 240
- US-A1- 2004 043 030
- WANG SHYH-JEN ET AL: "Intratumoral injection of rhenium-188 microspheres into an animal model of hepatoma" JOURNAL OF NUCLEAR MEDICINE, Bd. 39, Nr. 10, Oktober 1998 (1998-10), Seiten 1752-1757, XP002337420 ISSN: 0161-5505 in der Anmeldung erwähnt
- SAILEROVA E ET AL: "A study of factors affecting the labelling of tartrate with <188>Re and the transchelation of the <188>Re from the tartrate to a protein" APPLIED RADIATION AND ISOTOPES, PERGAMON PRESS LTD., EXETER, GB, Bd. 59, Nr. 5-6, November 2003 (2003-11), Seiten 311-319, XP004471909 ISSN: 0969-8043
- DU^A J ET AL: "Radiolabeling of dextran with rhenium-188" APPLIED RADIATION AND ISOTOPES, PERGAMON PRESS LTD., EXETER, GB, Bd. 53, Nr. 3, September 2000 (2000-09), Seiten 443-448, XP004204475 ISSN: 0969-8043
- DADACHOVA E ET AL: "The Role of Tin in the Direct Labelling of Proteins with Rhenium-188" NUCLEAR MEDICINE AND BIOLOGY, ELSEVIER SCIENCE PUBLISHERS, NEW YORK, NY, US, Bd. 24, Nr. 6, August 1997 (1997-08), Seiten 605-608, XP004089536 ISSN: 0969-8051
- WUNDERLICH G ET AL: "A kit for labeling of [<188>Re] human serum albumin microspheres for therapeutic use in nuclear medicine" APPLIED RADIATION AND ISOTOPES, PERGAMON PRESS LTD., EXETER, GB, Bd. 62, Nr. 6, Juni 2005 (2005-06), Seiten 915-918, XP004810524 ISSN: 0969-8043

## Beschreibung

Die Erfindung betrifft ein Verfahren zur Herstellung von mit dem radioaktiven Isotop Rhenium-188 (Re-188) markierten Partikeln, sowie einen Kit zur Durchführung des Verfahrens. Derart radioaktiv beladene Partikel können in der Medizin, vorzugsweise im Bereich der Onkologie und Nuklearmedizin, zur Radiotherapie von Tumoren oder Metastasen von Tumoren eingesetzt werden.

Die Radiotherapie von Tumoren oder deren Metastasen mit radioaktiv markierten Partikeln ist bekannt. Dazu wird in der Regel in die zu dem Tumor führenden Gefäße ein Katheter eingelegt. Durch den Katheter werden daraufhin die radioaktiv markierten Partikel lokal dem Tumorgewebe zugeführt. Die radioaktiv markierten Partikel haben eine Größe, die garantiert, dass sie bei einer ersten Passage des tumorinfiltrierenden Blutkapillarsystems in den Kapillaren des Tumors stecken bleiben. Die Methode erlaubt das Erreichen sehr hoher Radioaktivitätsdosen im Zieltumorgewebe bei gleichzeitiger Schonung des umgebenden Gewebes bzw. von anderen Organen im Patienten. Es werden wesentlich höhere Strahlendosen im Tumorgewebe erreicht, als zum Beispiel bei der systemischen intravenösen Applikation von radioaktiv markierten Antikörpern, Peptiden und anderen niedermolekularen Verbindungen.

Im letzten Jahrzehnt wurden vor allem die Radionuklide Y-90, Re-188 und Ho-166 zur Markierung von entsprechenden Partikeln eingesetzt. Der Betastrahler Re-188 mit der relativ geringen Halbwertszeit von 17 h eignet sich für eine Therapie mit hohen Radionukliddosen und mehrfacher Applikation am gleichen Patienten besonders gut.

Allerdings sind die bisherigen Markierungsverfahren für Re-188 und Partikel unbefriedigend.

Markierungsverfahren, die bei verwandten chemischen Elementen, wie z. B. Technetium effektiv durchführbar sind, sind auf Grund des unterschiedlichen chemischen Verhaltens, insbesondere durch das unterschiedliche Redoxpontial, auf Markierungen mit Re-188 nicht übertragbar.

Bevorzugte in der Nuklearmedizin als Trägermaterial für den Radionuklidtransport verwendete Partikel sind Humanserum Albumin-Mikrosphären einer mittleren Größe von 20 Mikrometern ([99mTc] HSA Mikrosphären B20, Rotop Pharmaka, Deutschland; Wunderlich G et al., Applied Radiation and Isotopes 52 (2000) S. 63-68). Diese Proteinpartikel sind im Organismus abbaubar, so dass die Mikrosphären die Kapillaren nur zeitweise verschließen und mehrfach dem Patienten infundiert werden können. Verwendet man das für Technetium entwickelte Markierungsverfahren zur Markierung mit Re-188 werden aufgrund der Unterschiede im Redoxpotential nur Ausbeuten für die Markierung unter 5 % erreicht.

Nachteilig ist bei dem von Wunderlich et al. beschriebenen Verfahren, dass nach über 90 Minuten Reaktionszeit nur 70 % bis maximal 90 % des Re-188 an die Partikel gebunden sind. Damit nicht umgesetztes Re-188 nicht zu einer unerwünschten Strahlenbelastung im Organismus des Patienten führt, muss überschüssiges Re-188 durch mehrfaches Waschen entfernt werden. Diese Waschschritte erfordern einen direkten Umgang mit radioaktiven Flüssigkeiten und haben daher eine hohe Strahlenbelastung für das Personal zu Folge.

In Veröffentlichungen von Wang S. J. et al (Journal of Nuclear Medicine 1998, 39 (10): S. 1752 -1757, Nuclear Medicine Communications 1998, 19: S. 427 - 433) sind ebenfalls Verfahren zur Markierung von Re-188 markierten Mikrosphären bekannt. Diese Mikrosphären bestehen aus einem Kunststoff Harz. Nachteilig müssen bei diesen Verfahren die Mikrosphären nach der Beladung mit Re-188 ebenfalls durch Entfernen des Überstandes und Resuspension in Kochsalzlösung gewaschen werden. Bei diesen Verfahren sind zur Markierung von 20 mg Mikrosphären 200 mg Zinnsalz und ein stark saurer pH-Wert nötig. Die hohe Zinnmenge hat den Nachteil, dass der Patient zusätzlich pharmakologisch belastet wird. Durch den starksauren pH-Wert ist das Verfahren für Proteinpartikel nicht geeignet, da das Protein durch die verwendete starksaure 0,2 N HCl hydrolysiert würde.

Grillenberger K. G. et al. beschreiben Re-188 markiertes Hydroxyapatit und Schwefelcolloid (Nuklearmedizin 1997, 36: S. 71 - 75). Die bei der Markierung erreichte Ausbeute ist allerdings unter 80 %.

Die bekannten Verfahren zur Markierung von Partikeln mit Re-188 sind zeitaufwendig. Die erzielten Markierungsausbeuten sind stark abhängig vom eingesetzten Basismaterial.

Es besteht in der Nuklearmedizin ein Bedarf an einem Verfahren, welches die Markierung von Partikeln mit Re-188 für das klinische Personal vereinfacht. Der Umgang mit hohen Radioaktivitätsdosen von Rhenium-188 sollte möglichst kurz sein, um die Strahlenbelastung für das Personal in einem vertretbaren Umfang zu halten. Ein Verfahren, dass auf Waschschritte verzichten kann, wäre daher erstrebenswert.

Aufgabe der Erfindung ist es, ein vereinfachtes Verfahren zur Markierung von Partikeln mit Rhenium-188, sowie ein Kit zur Durchführung des Verfahrens anzugeben. Insbesondere soll mit dem Verfahren und dem Kit die Strahlenbelastung des Personals und der Zeitaufwand bei der Durchführung des Verfahrens reduziert werden.

Erfindungsgemäß wird die Aufgabe durch ein Verfahren zur Herstellung von Rhenium-188 (Re-188) markierten Partikel gelöst, bei dem die Partikel zunächst in einer sauren Lösung suspendiert und erhitzt werden und nach einer gewissen Zeit des Erhitzens der pH-Wert erhöht wird.

Die Lösung hat dabei einen pH-Wert von pH 1 bis pH 3 und enthält:
a) ein Zinn-II-Salz und
b) ein Re-188-Perrhenatsalz.

Das bevorzugte Reaktionsvolumen beträgt in diesem Schritt 1 bis 5 ml, besonders bevorzugt 2 ml bis 4 ml, besonders vorteilhaft 3 ml. Die bekannten Re-Generatoren liefern ein Mindestelutionsvolumen von 2 ml. Vorteilhaft kann durch das Reaktionsvolumen im erfindungsgemäßen Verfahren das gesamte Eluat eines Re-Generators ausgenutzt werden.

Nach 30 bis 240 Minuten, vorzugsweise 45 bis 70 Minuten erhitzen wird der pH-Wert erhöht. Dabei wird der pH-Wert größer pH 5, bevorzugt zwischen pH 6,5 bis pH 8,5 eingestellt.

Erstaunlicherweise erhöht sich die Ausbeute der Markierung der Partikel mit Re-188 durch die Erhöhung des pH-Werts am Ende des Erhitzens auf über 95 %. Durch die so erreichte effektive Markierung der Partikel mit Re-188 ist eine weitere Aufarbeitung des Endprodukts nicht mehr notwendig. Insbesondere kann auf Waschschritte verzichtet werden. Die nach Erhöhung des pH-Werts erhaltene Suspension kann direkt zur Radiotherapie am Patienten eingesetzt werden.

Die Gesamtreaktionszeit verkürzt sich deutlich gegenüber dem Stand der Technik. Durch die wegfallenden Waschschritte wird neben der Zeitersparnis der Strahlenschutz für das Personal erheblich verbessert, da weniger Manipulationen notwendig sind, um ein injizierbares Produkt zu erreichen.

Mit dem Verfahren wird eine spezifische Radioaktivität (Beladung der Partikel) erreicht, die wesentlich über der Beladung liegt, die von Wunderlich et al. (2001) zuvor beschrieben wurde: 2500 MBq/mg gegenüber 500 MBq/mg.

Die Erhöhung des pH-Werts geschieht durch Zugabe einer Pufferlösung vorzugsweise eine Acetat, Citrat oder Tatratlösung, besonders vorzugsweise einer Kalium-Natriumtartrat Lösung.

Die Pufferlösung hat nach Zugabe zu der erhitzten Lösung bevorzugt eine Endkonzentration von 15mmol/l bis 50 mmol/l, besonders vorzugsweise 25 mmol/l.

Das Zinn-II-Salz ist vorzugsweise ein wasserlösliches Zinn II-Salz, wie z. Bsp. SnCl₂ x 2 H₂O oder SnF₂, welches zu Beginn des Verfahrens in der Lösung in einer Konzentration von 10 mmol/l bis 50 mmol/l vor, besonders vorzugsweise 17 mmol/l vorliegt.

Durch das Verfahren wird das zunächst als Perrhenat (ReO₄) in der Oxidationstufe +VII vorliegende Re-188 durch die reduktive Wirkung des Zinn-II-Salzes reduziert. Dadurch wird das Oxid des Re-188 in der Oxidationstufe +4 (ReO₂ x H₂O) zusammen mit dem sich bildenden schwerlöslichen Zinnhydroxid auf den Mikrosphären abgeschieden. Die so durch Coprecipation entstehende Schicht hat eine Dicke von circa 1 µm.

Durch das erfindungsgemäße Verfahren kann die Menge des zur Markierung nötigen Zinn-II-Salzes gegenüber dem Stand der Technik (Wang et al.) um Faktor 10 reduziert werden. Eine Menge von 10 mg bis 12 mg Zinn(II)Salz pro 10 mg Mikrosphären hat sich erstaunlicherweise als ausreichend für eine Beladung der Mikrosphären erwiesen.

Da Zinn-II-Salze in wässriger Lösung beim Erhitzen relativ instabil sind, wird der Lösung ein das Zinn-II-Salz stabilisierender Komplexbildner beigesetzt. Ein solcher Komplexbildner ist vorzugsweise eine organische Carbonsäure, besonders bevorzugt 2,5-Dihydroxybenzoesäure (Gentisinsäure). Weitere bevorzugte Komplexbildner sind Essigsäure, Zitronensäure, Malonsäure, Gluconsäure, Milchsäure, Hydroxyisobuttersäure, Ascorbinsäure, Weinsäure, Bernsteinsäure, die Salze der vorgenannten Säuren, oder Glucoheptonat. Der das Zinn-II-Salz stabilisierende Komplexbildner hat in der Lösung vorzugsweise eine Konzentration von 15 mmol/l bis 30 mmol/l, besonders vorzugsweise 20 mmol/l.

Die Verwendung von Gentisinsäure ist vorteilhaft, weil Gentisinsäure ein Radikalfänger ist und daher strahlenprotektiv im Präparat wirkt. Außerdem ist Gentisinsäure als Arzneimittelzusatzstoff bereits zugelassen.

Das Erhitzen der Lösung erfolgt vorzugsweise auf eine Temperatur unter dem Siedepunkt, in einem Bereich von 80 °C bis 100°C.

Die zu markierenden Partikel sind vorzugsweise kugelförmig bzw. annähernd rund. Derartige Partikel, Mikrosphären genannt, haben vorteilhaft einen Durchmesser, der klein genug ist, dass die Mikrosphären durch normale Blutgefäße transportiert werden, aber groß genug, dass sie in Kapillaren stecken bleiben. Vorzugsweise haben sie einen Durchmesser von 10 µm bis 100 µm, besonders vorzugsweise 15 µm bis 30 µm.

Die Partikel bestehen vorzugsweise aus einem organischen Polymer oder einem Biopolymer. In einer Variante der Erfindung bestehen die Partikel aus einem nicht *in vivo* abbabaubaren Polymer, bevorzugt einem schwachen Kationenaustauscherharz, (wie z. B. BioRex 70, BioRad, Deutschland), Polyacryl, Polymethylmethacrylat (PMMA, z. B. von Heraeus Kulzer, Deutschland), Methacrylatcopolymer (z. B. MacroPrep, BioRad, Gennany), oder Polyvinylformaldehyd (z.B. Drivalon Nycomed-Amersham, Deutschland).

Besonders bevorzugte Partikel sind jedoch Mikrosphären aus einem Material, das im menschlichen Organismus metabolisiert und abgebaut wird, so dass die Partikel die Kapillaren nach der Applikation nur zeitweise verschließen. Vorteilhaft wird dadurch eine mehrmalige Applikation der Partikel möglich. Ein bevorzugtes Beispiel für derartige abbaubare Partikel sind Mikrosphären aus humanem Serumalbumin ([^{99m}Tc] HSA Mikrosphären B20, Rotop Pharmaka, Radeberg, Deutschland). Die [^{99m}Tc] HSA Mikrosphären B20 sind für die Anwendungen mit einer Markierung durch Technetium-99m bereits zugelassen.

Vergleichsversuche mit Partikeln aus verschiedenen biodegradierbaren Materialien zeigten, dass sich mit Mikrosphären aus Humanem Serum Album in dem erfindungsgemäßen Verfahren überraschenderweise eine deutlich höhere Re-188 Markierungsausbeute und größere *in vivo* Stabilität erreichen lässt, als mit anderen Materialien, die ebenfalls *in vivo* abgebaut werden. So war die Markierungsausbeute mit Partikeln aus makroaggregiertem Albumin (MAA, Nycomed-Amersham, Deutschland), Kollagenpartikeln (Angiostat, Regional Therapeutics, USA) und Polylactat-Parikeln (PLA, Micromod, Deutschland) deutlich niedriger.

Die Partikel liegen bei der Markierung vorzugsweise in einer Konzentration von 2 bis 3 Millionen Partikeln, bevorzugt 2,5 Millionen Partikel, pro Milliliter bzw. 0,5 bis 10 Millionen Partikel pro Milliliter vor.

Der zur Markierung verwendete Betastrahler Rhenium-188 steht nach der Anschaffung eines entsprechenden Radionuklidgenerators (Oak Ridge National Laboratory, TN, USA oder Schering AG, Deutschland) praktisch unbegrenzt über mehrere Monate zur Verfügung und eignet sich besonders für eine Therapie mit hohen Radionukliddosen und mehreren Anwendungen am gleichen Patienten. In einem derartigen Generator wird das Re-188 durch Aufgabe einer 0,9% Kochsalzlösung in Form von Perrhenat (Oxdationstufe VII des Re-188) eluiert. Das so erhaltene Re-188-Generatoreluat hat vorzugsweise eine Radioaktivität von 1000 MBq bis 60000 MBq, vorzugsweise von 10000 bis 20000 MBq.

Die mit dem erfindungsgemäßen Verfahren erhaltene spezifische Radioaktivität (Beladung der Partikel) liegt vorzugsweise bei 1500 bis 3000 MBq/mg. Vorteilhaft kann die spezifische Radioaktivität patientenbezogen durch die eingesetzte Menge des Re-188-Generatoreluats auf die gewünschte therapeutische Strahlendosis eingestellt werden.

Vorteilhaft eignet sich das erfindungsgemäße Verfahren damit zur Markierung von Mikrosphären mit Radioaktivitäten, die im therapeutischen Bereich liegen. Dadurch und durch die genannte Vereinfachung der Verfahrensschritte wird vorteilhaft die Entwicklung eines pharmazeutischen Kits möglich.

Ein weiterer Gegenstand der Erfindung ist ein pharmazeutischer Kit zur Durchführung des erfindungsgemäßen Verfahrens. Dieser Kit zur Herstellung von Rhenium-188 markierten Mikrosphären enthält folgende Komponenten:
a) einen Behälter mit einer Menge eines wasserlöslichen Zinn-II-Salzes und eines Zinn-II-Salz stabilisierenden Komplexbildners, jeweils in Pulverform oder Lösung,
b) einen zweiten Behälter mit Mikrosphären aus humanem Serumalbumin, sowie
c) einen dritten Behälter mit einer Substanz oder Lösung zur Erhöhung des pH-Wertes in Pulverform oder Lösung.

Die Substanz zur Erhöhung des pH-Wertes liegt in fester oder wässriger Lösung vor und ergibt in Lösung einen pH-Wert von pH 6,5 bis pH 8,5.

Bevorzugt sind die Komponenten auf unterschiedliche Behälter verteilt. Der Kit enthält in dieser Ausführungsform mindestens je einen der drei Behälter pro Applikation am Patienten.

In einer besonders vorteilhaften Ausgestaltung der Erfindung wird zur Erhöhung des pH-Wertes Acetat, Citrat oder Tatrat, bevorzugt Kalium-Natriumtartrat verwendet. Pro Applikation am Patienten enthält der Kit vorzugsweise 0,1 mmol bis 0,2 mmol einer Substanz zur Erhöhung des pH-Werts, besonders vorzugsweise 30 mg bis 50 mg Kalium Natriumtartrat x 4 H₂O.

Das Zinn-II-Salz ist vorzugsweise ein wasserlösliches Zinn-II-Salz, wie z. Bsp. Zinn(II)chlorid-Dihydrat oder SnF₂. Pro Applikation am Patienten enthält der Kit vorzugsweise 0,02 mmol bis 0,1 mmol des wasserlöslichen Zinn-II-Salzes, besonders vorzugsweise 5 mg bis 20 mg Zinn(II)chlorid-Dihydrat

Da Zinn-II-Salze in wässriger Lösung beim Erhitzen relativ instabil sind, enthält der Kit bevorzugt als weitere Komponente einen das Zinn-II-Salz stabilisierenden Komplexbildner. Ein solcher Komplexbildner ist vorzugsweise eine organische Carbonsäure oder ein Salz einer organischen Carbonsäure. Der Komplexbildner ist in dem Behälter (a.) mit dem Zinn-II-Salz enthalten.

Ein besonders bevorzugter Zinn-II-Salz stabilisierender Komplexbildner ist 2,5-Dihydroxybenzoesäure (Gentisinsäure). Weitere bevorzugte Komplexbildner sind Acetat, Citrat, Malonat, Gluconat, Malat, Lactat, Hydroxyisobutyrat, Pyrophosphat, Ascorbat, Kalium-Natriumtartrat oder Glucoheptonat. Pro Applikation am Patienten enthält der Kit vorzugsweise 0,5 bis 2 Mol, besonders vorzugsweise 1 Mol, des Zinn-II-Salzstabilisierenden Komplexbildners pro Mol Zinn-II-Salz. Dies entspricht einer Menge von 5 mg bis 20 mg Gentisinsäure.

Der Kit enthält als weitere Komponente auch die zu markierenden Partikel. Diese Partikel sind vorzugsweise rund bzw. annähernd rund. Derartige Partikel, Mikrosphären, haben vorteilhaft einen Durchmesser, der klein genug ist, dass die Mikrosphären durch normale Blutgefäße transportiert werden, aber groß genug, dass sie in Kapillaren stecken bleiben. Vorzugsweise haben sie einen Durchmesser von 10 µm bis 50 µm, besonders vorzugsweise 10 µm bis 30 µm.

In dem Kit sind bevorzugt 0,5 bis 10 Millionen, besonders bevorzugt 1 bis 5 Millionen Partikel, vorteilhaft 1 bis 2 Millionen, in einem zusätzlichen Behälter (b.) enthalten.

Die Partikel bestehen vorzugsweise aus einem Material, das im menschlichen Organismus metabolisiert und abgebaut wird, so dass diese Partikel die Kapillaren bei der Anwendung nur zeitweise verschließen. Vorteilhaft wird dadurch eine mehrmalige Applikation der Partikel möglich. Ein bevorzugtes Beispiel für derartige abbaubare Partikel sind Mikrosphären aus humanem Serumalbumin ([^{99m}Tc] HSA Mikrosphären B20 Rotop Pharmaka, Radeberg, Deutschland). Die [99^{m}Tc] HSA Mikrosphären B20 sind für die Anwendungen mit einer Markierung mit Technetium-99m bereits zugelassen.

Die Partikel sind in dem Kit bevorzugt in einer konzentrierten wässrigen oder alkoholischen Suspension enthalten. Um die Dispersionsfähigkeit der Partikel zu erhöhen wir dieser Suspension vorteilhaft ein nichtionisches Detergens zugesetzt. Bevorzugt werden nichtionische Detergentien vom Polyethylen-Typ, wie Polyoxyethylensorbitanmonooleat (Tween® 80) verwendet.

Das nichtionische Detergens ist bevorzugt in einer Menge von 0,15 mg bis 0,3 mg pro 1 mg Partikel in der Suspension enthalten.

Zur Herstellung von Rhenium-188-markierten Mikrosphären wird das Zinn-II-Salzes und der Zinn-II-Salz stabilisierenden Komplexbildner im ersten Behälter in sterilem Wasser gelöst und zu dem zweiten Behälter mit den Mikrosphären gegeben und die Mikrosphären in der Lösung suspendiert. Zu der Suspension wird nun das radioaktive Rhenium-188-haltige Generatoreluat gegeben und die Suspension bei 80 bis 100 °C erhitzt. Nach 45 Minuten bis 70 Minuten Erhitzen wird durch Vermischen der Suspension mit der im dritten Behälter befindlichen Substanz zur Erhöhnung des pH-Werts der pH-Wert auf pH 5 bis pH 8,5 eingestellt. Die Suspension wird nun, bevorzugt auf Körpertemperatur, abgekühlt und kann ohne Waschschritte direkt dem Patienten verabreicht werden.

Die Erfindung betrifft auch die mit dem erfindungsgemäßen Verfahren bzw. dem erfindungsgemäßen Kit hergestellten Partikel und deren Verwendung zur Herstellung eines Mittels zur Radiotherapie von Karzinomen oder deren Metastasen.

Ein weiterer Bestandteil der Erfindung ist ein Verfahren zur Herstellung eines Mittels zur Radiotherapie von Tumoren, Karzinomen oder deren Metastasen mit diesen Partikeln. In dem Verfahren werden mit dem erfindungsgemäßen Verfahren wie oben beschrieben Re-188-markierte Partikel hergestellt. In das zu dem Karzinom führende lokale Blutgefäß wird ein Katheter eingelegt. Durch den Katheter wird daraufhin eine Suspension der radioaktiv markierten Partikel nach dem Einstellen des pH-Werts auf pH 5 bis pH 8,5 (ohne zwischengeschaltetes Waschen der Partikel) lokal dem Tumorgewebe zugeführt. Die radioaktiv markierten Partikel haben eine Größe, die garantiert, dass sie bei einer ersten Passage des tumorinfiltrierenden Blutkapillarsystems in den Kapillaren des Tumors stecken bleiben. Vorzugsweise haben die Partikel dazu einen Durchmesser von 10 µm bis 50 µm, besonders vorzugsweise 10 µm bis 30 µm.

Die Methode erlaubt vorteilhaft das Erreichen sehr hoher Radioaktivitätsdosen im Zieltumorgewebe bei gleichzeitiger Schonung des umgebenden Gewebes bzw. von anderen Organen im Patienten. Es werden wesentlich höhere Strahlendosen (100-150 Gy) im Tumorgewebe erreicht, als zum Beispiel bei der systemischen intravenösen Applikation von radioaktiv markierten Antikörpern, Peptiden und anderen niedermolekularen Verbindungen.

Die Verwendung von Mikrosphären aus Human-Serumalbumin hat den Vorteil, dass die Partikel im Körper abgebaut werden. Die Mikrosphären verschließen die Kapillaren bei der Applikation somit nur zeitweise. Eine mehrmalige Anwendung wird so ermöglich

Die Verabreichung der Partikel geschieht bevorzugt arteriell mittels Infusion. Bevorzugt werden dazu 0,5 bis 10 Millionen, besonders bevorzugt 1 bis 5 Millionen Partikel, vorteilhaft 1 bis 2, 5 Millionen (entsprechen 1 bis 20 mg, bevorzugt 3 bis 10 mg in 20 ml bis 100 ml, bevorzugt 50 ml, einer Infusionslösung (z. B. steriler isotonischer Kochsalzlösung) aufgeschwemmt und infundiert.

Die Mikrosphären werden mit einer biologischen Halbwertszeit im Bereich von bevorzugt größer 200 h, bevorzugt 8 Tagen bis 15 Tagen, abgebaut. Die biologische Halbwertszeit der Mikrosphären ist damit im Bereich der biologischen Halbwertszeit von Re-188. Vorteilhaft wird durch die Immobilisierung des Re-188 auf den Mikrosphären das Re-188 am Applikationsort fixiert (> 90% bleiben über Tage dort liegen).

Die mit dem erfindungsgemäßen Verfahren Re-188 markierten Mikrosphären eigen sich vorteilhaft insbesondere für die Therapie von Leberkarzinomen und Lebermetastasen anderer Karzinome.

Anhand der nachfolgenden Ausführungsbeispiele wird die Erfindung näher erläutert:

### Ausführungsbeispiel 1:

Die Markierung von Partikeln mit Re-188 wird anhand der Markierung von Human Serum Albumin (HSA) Mikrosphären wie folgt erläutert:
9,3 mg 2,5-Dihydroxybenzoesäure (Gentisinsäure) werden in 2 ml Wasser z. Injektion gelöst, anschließend werden 11,4 mg SnCl₂ H₂O zugegeben und die Lösung wird in eine Kit-Flasche mit Human Serum Albumin (HSA) Mikrosphären (MS B20, Rotop Pharmaka, Radeberg, Deutschland) steril filtriert. Die Partikel in der Flasche werden aufgeschlämmt und in eine weitere Kit-Flasche MS B 20 und anschließend in eine dritte Kit-Flasche überführt. In der dritten Flasche sind dann 1,5 Mio Partikel MS B 20 enthalten. Dazu wird 1 ml steril filtriertes Re-188 Perrhenat (10000-20000 MBq), gelöst in 0,9 % NaCl, gegeben. Die Kit-Flasche mit den Partikeln wird in einen Heizblock gestellt und dieser wird 55 Minuten bei 95 °C geschüttelt. Danach werden 0,6 ml steril filtrierte KNa-Tartrat-Lösung (42 mg/ml) zugegeben und die Heizung wird abgeschaltet. Nach 5 Minuten weiterem Schütteln ist das Präparat injektionsbereit.

Die Markierungsausbeute (radiochemische Reinheit) der so markierten Partikel beträgt ≥ 95%.

### Ausführungsbeispiel 2:

Ein bevorzugtes Kit zur Markierung von Partikeln (hier Human Serum Albumin (HSA) Mikrosphären) mit Re-188 bestehend aus drei Flaschen mit den in Tabelle 1 angebenden Inhaltstoffen:

**Tabelle 1 :**

| Flasche | Komponente | Mengen/ Flasche | Prozess | Konsistenz |
|---|---|---|---|---|
| 1 | 2,5 Dihydroxybenzoesäure | 9,3 mg | Lyophilisiert | fest |
| | Zinn(II)chlorid-Dihydrat | 11,4 mg | | |
| | Reinststickstoff 5.0 | | | |
| 2 | HSA Mikrosphären A20 (mit 10- 30 µm Durchmesser) | 10 mg (1,2 x 10⁶ bis 2 x 10⁶ Partikel) | Vakuumkonzentriert | fest |
| | Tween® 80 | 2,4 mg | | |
| | Reinststickstoff 5.0 | | | |
| 3 | Kalium-Natriumtartrat-Lösung (42 mg/ ml) | 1 ml | sterilisiert | flüssig |

| | | | | |
|---|---|---|---|---|
| **Reinststickstoff wird als Schutzgas verwendet | | | | |

Der Kit ist für eine Behandlung eines Patienten konzipiert.

### Ausführungsbeispiel 3:

Mit dem Kit gemäß Ausführungsbesipiel 2 werden die Partikeln (hier Human Serum Albumin (HSA) Mikrosphären) nach folgender Markierungsvorschrift markiert:

Die Bestandteile der Kitflasche 1 (2,5 Dihydroxybenzoesäure - Gentisinsäure) und Zinn(II)chlorid-Dihydrat) werden mit 2 ml sterilen, pyrogenfreien Wasser für Injektionszwecke gelöst und in Kitflasche 2 zu den HSA Mikrosphären A20 zugegeben. Nach Zugabe der Lösung ist zum Druckausgleich das gleiche Volumen an Stickstoff mit der Spritze den Fläschchen 1 und 2 zu entnehmen. Durch leichtes Schütteln unter Benetzung des Gummilyostopfens werden die HSA Mikrosphären suspendiert.
[¹⁸⁸Re]Natriumperrhenat in steriler, isotonischer, pyrogenfreier Natriumchloridlösung (¹⁸⁸Re-Generatoreluat, (10000-20000 MBq), Volumen: 1 ml) wird in das Fläschchen 2 überführt, welches sich in der Bleiabschirmung befindet. Nach Zugabe des ¹⁸⁸Re-Generatoreluates ist zum Druckausgleich das gleiche Volumen an Stickstoff aus dem Fläschchen 2 zu entnehmen.

Zur Reaktion wird das Fläschchen 2 in einem Heizschüttler 55 Minuten bei 95°C geschüttelt. Das Fläschchen 2 wird aus dem Schüttler entnommen und es werden 0,6 ml aus Fläschchen 3 (K/Na-Tartrat-Lösung) in Fläschchen 2 überführt. Nach Zugabe der Lösung ist zum Druckausgleich das gleiche Volumen an Stickstoff aus dem Fläschchen 2 zu entnehmen. Durch leichtes Schütteln unter Benetzung des Gummilyostopfens werden die [¹⁸⁸Re] HSA Mikrosphären suspendiert.
Das Präparat in Fläschchen reagiert weitere 5 min bei Raumtemperatur unter Verwendung eines Schüttelgerätes, wonach das Präparat injektionsfertig ist. Die Suspension der markierten [¹⁸⁸Re] HSA Mikrosphären B20 kann je nach gewünschter Konzentration mit Natriumchloridlösung zur Injektion verdünnt werden. Die [¹⁸⁸Re] HSA Mikrosphärensuspension ist bis 2 h nach der Markierung verwendbar.

### Ausführungsbeispiel 4:

Das Kit gemäß Ausführungsbeispiel 2 wird wie folgt hergestellt:

Für eine Charge von 150 Flaschen Nr. 1 werden 1,395 g Gentisinsäure (2,5 Dihydroxybenzoesäure) und 1,710 g Zinn(II)chlorid-Dihydrat in 150 ml Wasser für Injektionszwecke gelöst. Die Lösung wird auf 150 Flaschen verteilt und lyophilisiert.

Für eine Charge von 200 Flaschen Nr. 2 werden 2,0 g HSA Mikrosphären A20 (Rotop Pharmaka GmbH, Deutschland) und 0,48 g Tween® 80 in einer Lösung aus:
- 360 ml Aceton
- 40 ml Natronlauge (0,1 mol/l)
- 40 ml Salzsäure (0,1 mol/l)
- 240 ml Ethanol abs.
suspendiert. Der Suspension wird eine geringe Menge des Farbstoffes Bengalrosa zugesetzt. Die Suspension wird im Vakuum auf 400 ml konzentriert und auf die 200 Flaschen verteilt. Anschließend wird durch Vakuumtrocknen das Aceton und der Ethanol entfernt.

Für eine Charge von 150 Flaschen Nr. 3 werden 6,3 g KaliumNatriumtartrat in 150 ml Wasser für Injektionszwecke gelöst. Die Lösung wird auf 150 Flaschen verteilt.

### Ausführungsbeispiel 5:

Entsprechend der Vorschrift aus Ausführungsbeispiel 1 wurden Partikel aus verschiedenen Materialien mit Re-188 markiert:
- S1: Schwaches Polyacryl-Kationenaustauscherharz (Bio-Rex 70, BioRad, Deutschland),
- S2: Polymethylmethacrylat (PMMA, Heraeus Kulzer, Deutschland),
- S3: Methacrylatcopolymer (MacroPrep Q, BioRad, Deutschland),
- S4: Polyvinylformaldehyd (Drivalon Nycomed-Amersham, Deutschland),
- S5: MakroaggregiertemAlbumin (MAA, Nycomed-Amersham, Deutschland),
- S6: Humanes Serum Albumin (HSA B20, ROTOP Pharmaka GmbH, Deutschland),
- S7: Kollagenpartikel (Angiostat, Regional Therapeutics, USA),
- S8: Polylactat-Parikel (PLA, Micromod, Deutschland).

Eingesetzt wurden jeweils 2-3 mg der Partikel (entsprechen circa 0,5 Millionen Partikel).

Vor und nach der Markierung wurde die Verteilung der Partikelgröße mit Einzel-Partikel-Lichtstreuung gemäß ISO 13323-1 bestimmt. Nach Verdünnung in Partikel-freiem Wasser wurden die Partikel nacheinander in der Messzone der Durchflussküvette gemessen. Die Größenverteilung der Partikel wurde gemäß ISO 9276-2 in eine flächenbasierte Verteilung umgerechnet, da dies die Verteilung des Re-188 auf der markierten Partikeloberfläche besser charakterisiert.

In der Tabelle 2 sind die Werte der kumulativen Verteilung (Q₂) nach ISO 1998 angegeben, die Werte repräsentieren 90% der flächenbasierten Gesamtverteilung.

Die Markierungsausbeute wurde nach der Markierung durch Zentrifugieren der Teilchensuspensionen und Radioaktivitätsmessung von Überstand und Präzipitat in einem Gamma-Probenwechsler (Cobra II, Packard, USA) bestimmt.

**Tabelle 2:**

| | Material | | Partikelgröße vor Markierung [um] | Partikelgröße nach Markierung [µm] | Markierungsausbeute [%] |
|---|---|---|---|---|---|
| S1 | Biorex 70 | Makroreticulares Acrylharz | 45-75 | 30-75 | 80-85 |
| S2 | PMMA | Polymethylmethacrylat | 4-25 | 4-25 | 70-85 |
| S3 | Macro Prep | Methacrylatcopolymer | 45-55 | 30-80 | 83-90 |
| S4 | Drivalon | Polyvinylformaldehyd | 50-150 | 5-150 | 60-70 |
| S5 | MAA | Humanes Serumalbumin | 10-100 | 10-50 | 60-70 |
| S6 | HSA B20 | Humanes Serumalbumin | 13-27 | 15-37 | ≥ 95* |
| S7 | Angiostat | Kollagen | 20-75 | 1-15 | 35-50 |
| S8 | PLA | Polylactat | 10-45 | 3-45 | 50-60 |

Nach der Markierung mit Re-188 hatten die biodegradierbaren HSA Mikrosphären B20 (unter dem Mikroskop als Kugeln erkennbar) eine kaum veränderte Verteilung zwischen 15 µm und 37 µm, (Mittelwert 21 µm).
Im Gegensatz dazu hat makroaggregiertes HSA (MAA) nach der Markierung eine breitere Partikelverteilung. Das liegt daran, dass MAA-Partikel nicht als runde Mikrosphären, sondern irregulär etwa in der Art von Schwämmchen vorliegen. MAA-Partikel sind bei hohen Temperaturen nicht so stabil und werden aufgrund der größeren Oberfläche auch *in vivo* viel schneller enzymatisch angegriffen und abgebaut.

Drivalon (S4), Angiostat (S7) und PLA (S8) Partikel überstehen die Markierung bei der notwendigen hohen Reaktionstemperatur ebenfalls nicht gut, d.h., es tritt vermehrt Feinmaterial auf und die Partikelverteilung verbreitert sich signifikant. Trotzdem ist die Markierung bei allen Partikelpräparationen *in vitro* recht stabil.

Um die *in vitro* Stabilität zu testen, wurden die markierten Partikelproben mit humanem Plasma inkubiert. Nach dreistündiger Inkubation bei 37°C bzw. nach 24 und 48 stündiger Inkubation bei Raumtemperatur wurde die Anhaftung von Re-188 an den Partikeln nach Zentrifugation und Radioaktivitätsmessung in einem Gamma-Probenwechsler (Cobra II, Packard, USA) bestimmt.

Die Resultate zur *in vitro* Stabilität der markierten Partikel werden in Tabelle 3 zusammengefasst:

**Tabelle 3:**

| | Material | An Partikel gebundene Radioaktivität, Mittelwert ± Standardabweichung (SD) [%] | | | |
|---|---|---|---|---|---|
| | | t = 0 | t = 3h/37°C | t = 24h/22°C | t = 48h/22°C |
| S1 | Biorex 70 | 100 | 93,3±2,3 | 92,3±1,6 | 86,3±3,5 |
| S2 | PMMA | 100 | 95,2±1,7 | 93,8±2,4 | 91,3±2,7 |
| S3 | Macro Prep | 100 | 92,7±3,4 | 83,5±1,6 | 82,1±2,8 |
| S4 | Drivalon | 100 | 95,0±2,5 | 84,4±3,4 | 79,9±3,5 |
| S5 | MAA | 100 | 97,3±2,0 | 92,1±1,7 | 86,3±3,1 |
| S6 | HSA B20 | 100 | 98,0±1,8 | 92,2±2,2 | 86,8±2,4 |
| S7 | Angiostat | 100 | 92,0±3,4 | 85,2±3,2 | 82,0±1,6 |
| S8 | PLA | 100 | 96,1±2,7 | 80,4±2,8 | 75,7±1,9 |

Man kann die *in vitro* Stabilität aller Partikelpräparationen als ausreichend bezeichnen, da 75-90% des Re-188 nach 48h noch partikelgebunden vorliegt (Tabelle 3).

Die Bioverteilung der verschiedenen Partikel wurde *in vivo* nach intravenöser Injektion in Wistar-Ratten untersucht, wobei die Lunge als Modell für einen gut durchbluteten Tumor dient.

Nach Injektion von 20 MBq Re-188 markierten Partikeln wurde die Bioverteilung der Partikel über 48 Stunden unter der Gammakamera (Picker CX 250) mit Hilfe konventioneller nuklearmedizinischer Bildaufnahmetechnik untersucht. Nach Ende der Gammakamera-Untersuchungen wurden die Tiere getötet, ausgewählte Organe entnommen und deren Radioaktivität im Verhältnis zum Gesamttier und zur injizierten Aktivität in einem GammaZähler bestimmt.

Die in *vivo* Bioverteilung in Leber und Lunge (jeweils in % der injizierten Dosis im Gesamtorgan) der markierten Partikel wurde 48 h nach Injektion in die Schwanzvene der 8 Wochen alten Wistar-Ratten (n = 3 bis 6) pro Material bestimmt.

Um die in *vivo* Stabilität der unterschiedlichen Partikelpräparationen zu bestimmen wurde als Maß die biologische Halbwertszeit in der Lunge (T_{b} ½) verwendet, die Werte zwischen 45 h, bis zu über 200 h lieferte. Eine biologische Halbwertszeit von 200 h entspricht dabei einer effektiven Halbwertszeit von 15,4 h für Re-188. Da nach fünf effektiven Halbwertszeiten (d. h. 77 h) lediglich noch etwa 3% der ursprünglichen Radioaktivität im Körper vorhanden sind und therapeutisch wirken können, kann man die erreichte Stabilität als ausreichend bezeichnen.

Die Resultate der *in vivo* Bioverteilung und der *in vivo* Stabilität werden in Tabelle 4 zusammengefasst:

**Tabelle 4:**

| | Material | Leber (% injizierte Dosis) | Lunge (% injizierte Dosis) | T _{b1/2} [h] |
|---|---|---|---|---|
| S1 | Biorex 70 | 3,9 | 91,4 | > 200 |
| S2 | PMMA | 16,7 | 76,4 | >200 |
| S3 | Macro Prep | 0,9 | 85,1 | >200 |
| S4 | Drivalon | 56,5 | 19,6 | 125,3 |
| S5 | MAA | 2,8 | 48,0 | 45,4 |
| S6 | HSA B20 | 0,8 | 92,9 | >200 |
| S7 | Angiostat | 49,6 | 14,5 | 129,7 |
| S8 | PLA | 11,1 | 66,5 | 153,9 |

Die Bioverteilungsstudien zeigen sehr gute in vivo Stabilität für die Präparate S1- S3 und S6, charakterisiert durch sehr langsames Absinken der Radioaktivität in der Lunge und eine geringe Radioaktivitätsaufnahme in den Nicht-Ziel-Geweben (z.B. der Leber, außer bei S2 - Tabelle 4). S2 hat bereits im Basismaterial eine relative hohen Feinanteil, der zur Partikelanreicherung in der Leber führt, wo die Partikel jedoch ebenfalls über den Versuchszeitraum unverändert liegen bleiben.

Kleine Partikel (< 10 µm, wie z. B. Probe S2) werden im retikulo-endothelialen System (RES) in Leber und Milz gesammelt. Wenn sich Feinmaterial im Markierungsprozess bildet, wird es nach intravenöser (i.v.) Injektion in diesen Organen gefunden (Proben S4, S7 und S8). Diese Partikel eignen sich also nicht für die intraarterielle Tumortherapie in Menschen, obwohl die biologische Halbwertszeit relativ lang (>120h) ist.

MAA-Makroaggregate (S5) eignen sich wiederum wegen ihrer relative geringen biologischen Halbwertszeit (45,4h) nicht für die intraarterielle Tumortherapie in Menschen.

Verglichen mit den fatalen medizinischen Resultaten, über die Mantravadi 1981 (Mantravadi RV, Spigos DG, Tan WS, Felix EL. Intraarterial yttrium-90 in the treatment of hepatic malignancy. Radiology 1981;142:783-786.) nach der Anwendung eines nicht ausreichend fest an Partikel gebundenen längerlebigen Betaemitters (Y-90) berichtet, ist die Anwendung von Partikeln, die mit dem kurzlebigeren Strahler Re-188 markiert sind, erheblich weniger gefährlich. Von den Partikeln freigesetztes Re-188 wird nämlich nicht in lebenswichtigen Organen angereichert, sondern in kurzer Zeit über die Nieren ausgeschieden.
Ein weiterer Vorteil der Verwendung von Re-188 Präparaten ist, dass der vorhandene Radionuklidgenerator jederzeit zur Herstellung von Re-188 Präparaten verwendet werden kann, weshalb auf ärztliche Anforderungen ohne längere Bestellzeiten und mit attraktiven Kosten reagiert werden kann.

Die Resultate der Vergleichsversuche mit den verschiedenen Partikelmaterialien können, wie folgt zusammengefasst werden:

Mit dem erfindungsgemäßen Verfahren können verschiedene Partikelmaterialien in hoher Ausbeute mit Re-188 markiert und einer vielversprechenden Weise für endoradiotherapeutische Anwendungen angewandt werden.

HSA Microsphären B20 markiert mit Re-188 sind das attraktivste nuklearmedizinische Therapeutikum für eine lokale Tumortherapie nach selektiver Katheterisierung der zuführenden Blutgefäße., insbesondere wegen der Biokompatibilität der Partikel, ihrer einheitlichen Größe und wegen der hohen in vivo Stabilität des Produkts.

## Patentansprüche

1. Verfahren zur Herstellung von Rhenium-188 beladenen Partikeln bei dem Partikel aus einem organischen Polymer oder aus einem Biopolymer in einer Lösung suspendiert und auf 80 °C bis 100 °C erhitzt werden, wobei die Lösung zunächst einen pH-Wert von pH 1 bis pH 3 aufweist und enthält:
a.) ein wasserlösliches Zinn-II-Salz,
b.) ein Re-188 Perrhenatsalz mit einer Radioaktivität von 1000 MBq bis 60000 MBq,
**dadurch gekennzeichnet, dass** nach 45 Minuten bis 70 Minuten Erhitzen der pH-Wert erhöht und auf einen pH-Wert von pH 5 bis pH 8,5 eingestellt wird.

2. Verfahren nach Anspruch 1, **dadurch gekennzeichnet, dass** zur Erhöhung des pH-Werts eine Lösung aus Citrat, Acetat oder Tartrat, vorzugsweise Kaliumnatriumtartrat, verwendet wird.

3. Verfahren nach einem der Ansprüche 1 oder 2, **dadurch gekennzeichnet dass** die Lösung einen das Zinn-II-Salz stabilisierenden Komplexbildner, ausgewählt aus 2,5-Dihydroxybenzoesäure, Essigsäure, Zitronensäure, Malonsäure, Gluconsäure, Milchsäure, Hydroxyisobuttersäure, Ascorbinsäure, Weinsäure, Bernsteinsäure, den Salzen der vorgenannten Säuren, oder Glucoheptonat enthält.

4. Verfahren nach Anspruch 3, **dadurch gekennzeichnet, dass** als Zinn-II-Salz stabilisierender Komplexbildner 2,5-Dihydroxybenzoesäure verwendet wird.

5. Verfahren nach einem der Ansprüche 1 bis 4, **dadurch gekennzeichnet, dass** die Partikel einen Durchmesser von 10 µm bis 30 µm haben.

6. Verfahren nach einem der Ansprüche 1 bis 5, **dadurch gekennzeichnet, dass** das wasserlösliche Zinn-II-Salz zu Beginn des Verfahrens in der Lösung in einer Konzentration von 10 mmol/l bis 50 mmol/l vorliegt.

7. Verfahren nach einem der Ansprüche 1 bis 6, **dadurch gekennzeichnet, dass** die Partikel aus Humanem Serum Albumin bestehen.

8. Pharmazeutischer Kit zur Herstellung von Rhenium-188-markierten beladenen Partikeln enthaltend:
a.) einen ersten Behälter mit einer Menge eines wasserlöslichen Zinn-II-Salzes und einer Menge eines Zinn-II-Salz stabilisierenden Komplexbildners, ausgewählt aus 2,5-Dihydroxybenzoesäure, Essigsäure, Zitronensäure, Malonsäure, Gluconsäure, Milchsäure, Hydroxyisobuttersäure, Ascorbinsäure, Weinsäure, Bernsteinsäure, den Salzen der vorgenannten Säuren, oder Glucoheptonat,
b.) einen zweiten Behälter mit Partikeln aus einem organischen Polymer oder aus einem Biopolymer,
c.) einen dritten Behälter mit einer Menge einer Substanz zur Erhöhung des pH-Werts, ausgewählt aus Citrat, Acetat oder Tartrat, die in fester Form oder in wässriger Lösung vorliegt und in Lösung einen pH-Wert von pH 6,5 bis pH 8,5 ergibt.

9. Pharmazeutischer Kit nach Anspruch 8, **dadurch gekennzeichnet, dass** der Zinn-II-Salz stabilisierender Komplexbildner 2,5-Dihydroxybenzoesäure ist.

10. Pharmazeutischer Kit nach Anspruch 8 oder 9, **dadurch gekennzeichnet, dass** die Substanz zur Erhöhung des pH-Werts Kaliumnatriumtartrat ist.

11. Pharmazeutischer Kit nach einem der Ansprüche 8 bis 10, **dadurch gekennzeichnet, dass** die Partikel einen Durchmesser von 10 µm bis 30 µm haben.

12. Pharmazeutischer Kit nach einem der Ansprüche 8 bis 11, **dadurch gekennzeichnet, dass** der Kit pro Applikation am Patienten 0,02 mmol bis 0,1 mmol Zinn-II-Salz enthält.

13. Pharmazeutischer Kit nach einem der Ansprüche 8 bis 12, **dadurch gekennzeichnet, dass** die Partikel aus Humanem Serum Albumin bestehen.

14. Rhenium-188 beladene Partikel hergestellt mit dem Verfahren nach einem der Ansprüche 1 bis 6.

15. Verwendung von Rhenium-188 beladenen Partikeln nach Anspruch 14 zur Herstellung eines Mittels zur Radiotherapie von Tumoren, Karzinomen oder deren Metastasen.

## Claims

1. Method for producing rhenium-188 labeled particles in which particles of an organic polymer or a biopolymer are suspended in a solution and heated to 80 °C to 100 °C, wherein the solution has initially a pH value of pH 1 to pH 3 and comprises:
a) a water-soluble tin-II salt,
b) a Re-188 perrhenate salt with a radioactivity of 1,000 MBq to 60,000 MBq,
**characterized in that** after 45 minutes to 70 minutes of heating the pH value is increased and adjusted to a pH value of pH 5 to pH 8.5.

2. Method according to claim 1, **characterized in that** for increasing the pH value a solution of citrate, acetate, or tartrate, preferably potassium sodium tartrate, is used.

3. Method according to one of the claims 1 or 2, **characterized in that** the solution contains a complexing agent for stabilizing the tin-II salt, selected from 2, 5-dihydroxy benzoic acid, acetic acid, citric acid, malonic acid, gluconic acid, lactic acid, hydroxy isobutyric acid, ascorbic acid, tartaric acid, succinic acid, the salts of the aforementioned acids or glucoheptonate.

4. Method according to claim 3, **characterized in that** 2,5-dihydroxy benzoic acid is used as the complexing agent for stabilizing the tin-II salt.

5. Method according to one of the claims 1 to 4, **characterized in that** the particles have a diameter of 10 µm to 30 µm.

6. Method according to one of the claims 1 to 5, **characterized in that** the water-soluble tin-II salt at the beginning of the method is present in the solution in a concentration of 10 mmol/l to 50 mmol/l.

7. Method according to one of the claims 1 to 6, **characterized in that** the particles are comprised of human serum albumin.

8. Pharmaceutical kit for producing particles labelled with Re-188, comprising:
a.) a first container with a quantity of water soluble tin-II salt and a quantity of a complexing agent for stabilizing the tin-II salt, selected from 2,5-dihydroxy benzoic acid, acetic acid, citric acid, malonic acid, gluconic acid, lactic acid, hydroxy isobutyric acid, ascorbic acid, tartaric acid, succinic acid, the salts of the aforementioned acids or glucoheptonate;
b.) a second container with particles made from an organic polymer or a biopolymer;
c.) a third container with a quantity of a substance for increasing the pH value, selected from citrate, acetate, or tartrate, present in solid form or in aqueous solution and generating in solution a pH value of pH 6.5 to pH 8.5.

9. Pharmaceutical kit according to claim 8, **characterized in that** 2,5dihydroxy benzoic acid is the complexing agent for stabilizing the tin-II salt.

10. Pharmaceutical kit according to claim 8 or 9, **characterized in that** the substance for increasing the pH value is potassium sodium tartrate.

11. Pharmaceutical kit according to one of the claims 8 to 10, **characterized in that** the particles have a diameter of 10 µm to 30 µm.

12. Pharmaceutical kit according to one of the claims 8 to 11, **characterized in that** the kit contains 0.02 mmol to 0.1 mmol tin-II salt per administration to the patient.

13. Pharmaceutical kit according to one of the claims 8 to 12, **characterized in that** the particles are comprised of human serum albumin.

14. Rhenium-188 labeled particles produced by the method according to one of the claims 1 to 6.

15. Use of rhenium-188 labeled particles according to claim 14 for producing an agent for the radiotherapy of tumors, carcinoma or their metastases.

## Revendications

1. Procédé de production de particules chargées au rhénium 188, dans lequel des particules d'un polymère organique ou d'un biopolymère sont mises en suspension dans une solution et sont chauffées entre 80 °C et 100 °C, la solution présentant d'abord une valeur de pH comprise entre pH 1 et pH 3 et contenant :
a) un sel d'étain II hydrosoluble ;
b) un sel perrhénate de Re-188 présentant une radioactivité comprise entre 1 000 MBq et 60 000 MBq,
**caractérisé en ce qu'**après 45 à 70 minutes de chauffage, la valeur de pH est augmentée et est réglée à une valeur de pH comprise entre pH 5 et pH 8,5.

2. Procédé selon la revendication 1, **caractérisé en ce que** pour augmenter la valeur de pH, on utilise une solution de citrate, d'acétate ou de tartrate, de préférence de tartrate de potassium et de sodium.

3. Procédé selon l'une des revendications 1 ou 2, **caractérisé en ce que** la solution contient un agent complexant stabilisant le sel d'étain II sélectionné parmi l'acide 2,5-dihydroxybenzoïque, l'acide acétique, l'acide citrique, l'acide malonique, l'acide gluconique, l'acide lactique, l'acide hydroxyisobutyrique, l'acide ascorbique, l'acide tartrique, l'acide succinique, les sels des acides précités ou le glucoheptonate.

4. Procédé selon la revendication 3, **caractérisé en ce que** l'on utilise l'acide 2,5-dihydroxybenzoïque en tant qu'agent complexant stabilisant le sel d'étain II.

5. Procédé selon l'une des revendications 1 à 4, **caractérisé en ce que** les particules ont un diamètre compris entre 10 µm et 30 µm.

6. Procédé selon l'une des revendications 1 à 5, **caractérisé en ce que** le sel d'étain II hydrosoluble, au début du procédé, est présent dans la solution selon une concentration comprise entre 10 mmol/l et 50 mmol/l.

7. Procédé selon l'une des revendications 1 à 6, **caractérisé en ce que** les particules se composent de sérum-albumine humain.

8. Trousse ou ensemble pharmaceutique de production de particules chargées marquées au rhénium 188, contenant :
a) un premier récipient comportant une quantité d'un sel d'étain II hydrosoluble et une quantité d'un agent complexant stabilisant le sel d'étain Il sélectionné parmi l'acide 2,5-dihydroxybenzoïque, l'acide acétique, l'acide citrique, l'acide malonique, l'acide gluconique, l'acide lactique, l'acide hydroxyisobutyrique, l'acide ascorbique, l'acide tartrique, l'acide succinique, les sels des acides précités ou le glucoheptonate,
b) un second récipient contenant des particules d'un polymère organique ou d'un biopolymère,
c) un troisième récipient contenant une quantité d'une substance permettant d'augmenter la valeur du pH, sélectionnée parmi le citrate, l'acétate ou le tartrate, qui se présente sous une forme solide ou en solution aqueuse, et donne en solution une valeur de pH comprise entre pH 6,5 et pH 8,5.

9. Trousse pharmaceutique selon la revendication 8, **caractérisée en ce que** l'agent complexant stabilisant le sel d'étain II est l'acide 2,5-dihydroxybenzoïque.

10. Trousse pharmaceutique selon la revendication 8 ou 9, **caractérisée en ce que** la substance permettant d'augmenter la valeur de pH est le tartrate de potassium et de sodium.

11. Trousse pharmaceutique selon l'une des revendications 8 à 10, **caractérisée en ce que** les particules ont un diamètre compris entre 10 µm et 30 µm.

12. Trousse pharmaceutique selon l'une des revendications 8 à 11, **caractérisée en ce que** la trousse, par application sur un patient, contient 0,02 mmol à 0,1 mmol de sel d'étain II.

13. Trousse pharmaceutique selon l'une des revendications 8 à 12, **caractérisée en ce que** les particules se composent de sérum-albumine humain.

14. Particule chargée au rhénium 188 produite par le procédé selon l'une des revendications 1 à 6.

15. Utilisation de particules chargées au rhénium 188 selon la revendication 14 pour produire un agent destiné à la radiothérapie de tumeurs, de carcinomes ou de leurs métastases.
